(19) Europäisches Patentamt
European Patent Office
Office européen des brevets

(11) **EP 3 831 424 A1**

(12) **EUROPEAN PATENT APPLICATION**

(43) Date of publication:
**09.06.2021 Bulletin 2021/23**

(21) Application number: **19212856.9**

(22) Date of filing: **02.12.2019**

(51) Int Cl.:
**A61M 5/00** *(2006.01)*  **A61M 5/145** *(2006.01)*
**A61M 5/168** *(2006.01)*  *A61B 6/00* *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(71) Applicant: **Siemens Healthcare GmbH
91052 Erlangen (DE)**

(72) Inventor: **Gutjahr, Ralf
90459 Nürnberg (DE)**

(54) **CONTROLLER FOR A CONTRAST MEDIA POWER INJECTOR**

(57) The invention describes a controller (40) for a contrast media power injector (20, 20') which comprises a first vessel (22) filled with contrast medium (23). The concentration of the contrast medium (23) is higher than the required concentration for a first injection step (IV). Furthermore, the contrast media power injector (20, 20') comprises a second vessel (28) filled with saline solution (29). The controller (40) is realised to release controlled amounts of contrast medium (23) and saline solution (29) to obtain a combined solution with the concentration required for the first injection step (IV). The invention further describes a related method; contrast media power injector (20, 20') and an imaging system (100).

FIG 1

EP 3 831 424 A1

## Description

The invention describes a controller and a method for controlling a contrast media power injector, a contrast media power injector, and an imaging system.

Because of the similar appearance of different organs and, in general, different kinds of tissue in radiographic imaging, procedures like soft tissue and angiographic imaging in Computed Tomography (CT) or Magnetic Resonance Imaging (MRI) or ultrasonic imaging (US) rely on the application of intravenous injections of contrast media (CM).

Iodinated contrast medium, as most commonly used in contrast enhanced CT, provides an increased contrast within the arterial system (early phase) and organ parenchyma (later phase). Gadolinium, for example, is used for the same purpose in MRI.

Generally, the achieved contrast enhancement depends on the applied concentration of the contrast medium, the total volume of contrast medium (VCM), the injection duration (T), and the injection flow rate (FR) to which the relationship VCM = T * FR applies. Furthermore, scan parameters (i.e. tube voltage, scan timing) and patient characteristics (i.e. patient size, total blood volume, cardiac output) influence the contrast enhancement. Thus, adjusting the scan parameters and/or contrast injection parameters for the imaging procedure improves the imaging results.

The delivery rate of the contrast medium (DR), which is the product of the injection flow rate, the contrast medium concentration, and the contrast medium ratio, is an important variable that allows, for example, control of the magnitude of the arterial enhancement. In order to achieve an increased contrast enhancement, either the injection flow rate and/or the contrast medium concentration can be increased. For example, higher contrast medium concentrations are reportedly used for patients with underlying cardiocirculatory disease or to detect hypervascular lesions in the liver during CT perfusion imaging. In a few cases highly concentrated contrast medium can cause beam hardening artefacts impairing the ultimate diagnosis (like perivenous artefacts at the level of brachiocephalic veins and the superior vena cava in thoracic MD-CTA). Concentrations of contrast medium which are too high should, thus, preferably be avoided.

Yet, especially for newborns, children, and elderly, the risk of extravasation is increased when using high injection flow rates. In these scenarios the delivery rate can just be increased by applying highly concentrated contrast medium.

For later phases, however, the local deposited contrast medium, a function of total contrast medium dose and the size of an organ determines the contrast enhancement. Alternatively, the local enhancement is determined by the number of molecules of contrast medium within a volume. Thus, for later phases, a high delivery rate is not required and flow rates and/or contrast medium concentrations can be kept at a moderate level.

This might be favourable since some studies link contrast medium with high concentrations to toxic side effects, such as so-called contrast induced nephrotoxicity (CIN).

Until now, in order to provide the required concentration, it is necessary to change the containers for the contrast medium between different imaging procedures or steps thereof. This leads to a delay in logistics and may also have a negative impact on hygiene conditions.

It is an object of the present invention to improve the injection process of contrast media.

This object is achieved by a controller according to claim 1; a contrast media pressure injector according to claim 9; an imaging system according to claim 11; and a method according to claim 12.

According to the invention, the controller is for a contrast media power injector which comprises a first vessel filled with contrast medium. The concentration of the contrast medium is higher than the concentration required for a first injection step. A second vessel comprised by the power injector is filled with saline solution. The controller is realised to release controlled amounts of contrast medium and saline solution to obtain a combined solution with the concentration required for the first injection step.

A contrast medium power injector is also referred to as a contrast media injector, contrast power injector or radiology pressure injector or simply a power injector. It is realised to administer a contrast medium to an examination object, in particular an animal or human patient, during a radiographic imaging procedure such as X-ray imaging, CT, MRI, US etc.

In the context of the invention, a vessel means a container which is suited to be filled with the respective substance. In a simple embodiment the vessel can be realised as a plastic bag or the like.

The term contrast medium or contrast agent generally describes a medium that enhances the contrast and in this context a contrast enhancer for radiographic imaging. For example, iodinated contrast media are utilised for CT or X-ray imaging, while contrast agents comprising e. g. Gadolinium are employed in MRI and contrast agents comprising e.g. microbubbles of sulphur hexafluoride are utilised in US.

In order to mix the two components, i.e. contrast medium and saline solution, the two vessels are fluidically connected, e.g. via tubing and a coupling such as a three-way valve, a T-piece or the like. Furthermore, the two vessels can be fluidically connected to the examination object via additional tubing and a suited port or the like. This connection can be established by medical personnel, but is expressly not encompassed by the scope of the invention.

In the state of the art mixing contrast medium with saline solution only takes place in the later phase of the imaging procedure i.e. the organ phase. In contrast, the controller according to the invention regulates the dilution of the contrast medium already in the first injection step. The first injection step corresponds to the early phase of the imaging process. In other words, for one imaging procedure, there is no contrast injection step before the first contrast injection step.

This means that already in the early phases of the imaging process, i.e. the arterial phase, the highly concentrated contrast medium is diluted with the saline solution. Accordingly, the mixture of both highly concentrated contrast medium and saline solution form the combined i.e. mixed solution. Therefore, the concentration of the contrast medium in the first vessel must be higher than required for the arterial phase of the imaging procedure. The concentration of contrast medium in the combined solution utilised during the first injection step is also referred to as first effective concentration or first target concentration.

Due to the dilution of the highly concentrated contrast medium less volume of the contrast medium is utilised during the imaging procedure. Due to the arbitrarily adjustable dilution of the contrast medium, any lower concentration can be mixed for the injection step(s). Thus, advantageously, a single container with highly concentrated contrast medium can be utilised for different clinical diagnoses. This advantageously results in fewer exchanges of the vessel containing the contrast medium and facilitates logistics and improves hygiene conditions.

According to the invention, the contrast media power injector comprises a first vessel filled with contrast medium at a higher concentration than a concentration to be injected in a first injection step. It additionally comprises a second vessel filled with saline solution and a controller according to the invention. The same term definitions apply as stated above.

The imaging system according to the invention comprises an imaging modality and a contrast media power injector according to the invention. The imaging modality can for example be realised as an X-ray device, a CT device or a MRI device. The imaging modality is preferably connected to the power injector in order to coordinate the injection of contrast medium and the imaging process.

According to the invention, the method is provided for controlling a contrast media power injector which comprises a first vessel filled with contrast medium. The concentration of the contrast medium is higher than the required concentration for a first injection step. Furthermore, the contrast media power injector comprises a second vessel filled with saline solution. The inventive method comprises the step of releasing controlled amounts of contrast medium and saline solution to obtain a combined solution with the concentration required for the first injection step. The same definitions of terms apply as stated above in the context of the description of the controller.

Some units or modules of the controller mentioned above can be completely or partially realized as software modules running on a processor of a device. A realization largely in the form of software modules can have the advantage that applications already installed on an existing controller can be updated, with relatively little effort, to install and run these units of the present application. The object of the invention is also achieved by a computer program product with a computer program that is directly loadable into the memory of a device of a controller for a contrast media power injector, and which comprises program units to perform the steps of the inventive method when the program is executed by the controller. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a controller. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

The controller is preferably integrated in the power injector. The power injector is, therefore, a compact device that can advantageously operate in a standalone mode.

An alternative but also preferred controller is located separate from the power injector, but connected to the power injector. Via the connection, the controller sends control signals which control the release of contrast medium and saline solution. This embodiment of the controller is preferably integrated in the imaging modality or realised on a separate computing device. Advantageously, the power injector can, therefore, be controlled from a distance and a coordinated control of the power injector and the imaging modality is facilitated.

The controller is preferably designed to control the release of contrast medium and saline solution to a solution with a, preferably lower, concentration to be injected in a second injection step. The second injection corresponds in time to the later phase, i.e. organ phase, of the imaging procedure. The concentration of contrast medium in the combined solution in the second injection step is preferably lower than the concentration of contrast medium in the combined solution in the first injection step. The concentration of contrast medium in the combined solution utilised during the second injection step is also referred to as second effective concentration or second target concentration. This may advantageously yield in cost savings and a decreased risk of chronic nephropathy.

Preferably, the controller is designed to control the release of saline solution after the injection of the contrast medium. This means that after the first injection step or after the first and the second injection step only pure saline solution is released. This is also referred to as "saline chaser". The use of the saline chaser may advantageously yield in further cost savings and a decreased risk of chronic nephropathy. If the saline chaser is injected, as preferred, with the same total flow rate as in the preceding injection step(s), this prevents the previously injected combined solution of contrast medium and saline solution from phasing out during the imaging procedure. Thus, the contrast enhancement is improved and lasts longer.

It is preferred to keep the total flow rate constant for all injection steps.

In general, it is possible and encompassed by the invention to change the settings of control parameters, i.e. injection parameters, during the injection procedure and even during a specific injection step. However, the controller controls the release of contrast medium and saline solution preferably according to a predefined injection protocol. The injection protocol therefore preferably comprises a time sequence with control commands for all required injection steps. The injection protocol is based on injection parameters such as e.g. contrast medium ratio, injection flow rate, contrast medium delivery rate, contrast medium concentration. The release of the substances from the at least two vessels of the contrast media power injector is preferably controlled taking into account at least one of these parameters. It should be noted, that these parameters are determined separately for each injection step.

The injection protocol can, for instance, be predefined as a default protocol stored in the controller. The default protocol can preferably be modified by the medical personnel. Alternatively, for increased flexibility the injection protocol and values for its parameters can be predefined via a user input.

The contrast medium ratio can also be varied to achieve a desired (effective) concentration in the combined solution to adjust to patient size specific contrast scaling, while retaining the timing parameters of the injection protocol, and, therefore, also retaining its dynamics within the patient's circulatory system.

It is preferred to predefine the injection protocol according to patient parameters such as for example patient size, patient weight, patient age, patient sex, the indication to be examined etc. Individual injection protocols may be stored in the controller for different sets of values for the patient parameters. Alternatively, an injection protocol can be generated or modified by using look-up tables which link patient parameters to injection parameters or the injection parameters can be calculated from the patient parameters. This facilitates the set-up of the injection procedure.

As described above, generally, different substances can be utilised as contrast media. Preferably, the contrast medium is an iodinated contrast medium. This is especially advantageous for X-ray and/or CT imaging.

In embodiments of the invention the highest possible concentration of contrast medium in the first vessel of the contrast media power injector is preferred, since less volume of the contrast medium is required. However, the concentration value is limited by unfeasible viscosities at very high concentrations. In general, concentrations can be indicated in the form mg/ml. If the concentration indications refer to iodine, they are indicated in the following form mgI/mL. Thus, for instance a preferred concentration of iodine in the contrast medium is at least 350 mgI/mL, more preferably at least 370 mgI/mL, even more preferably at least 390 mgI/mL, mostly preferably at least 400 mgI/mL.

In a preferred embodiment of the invention, the first vessel and/or the second vessel are realised as syringes. For syringes the release of the contained liquid is controlled, for example, by a motor-driven forward feed of a plunger.

In an additional or alternative but also preferred embodiment of the invention the first vessel and/or the second vessel are realised as bottles. For bottles the release of the contained liquid is controlled, for example, by suited kinds of pumps.

Other objects and features of the present invention will become apparent from the following detailed descriptions considered in conjunction with the accompanying drawings. It is to be understood, however, that the drawings are designed solely for the purposes of illustration and not as a definition of the limits of the invention.

FIG 1 shows an exemplary simplified imaging system according to an embodiment of the invention;

FIG 2 shows an exemplary contrast media power injector system according to an embodiment of the invention;

FIG 3 shows a block diagram of an exemplary controller according to an embodiment of the invention;

FIG 4 shows a flow chart of an exemplary method for controlling a contrast media power injector according to an embodiment of the invention.

In the diagrams, like numbers refer to like objects throughout. Objects in the diagrams are not necessarily drawn to scale.

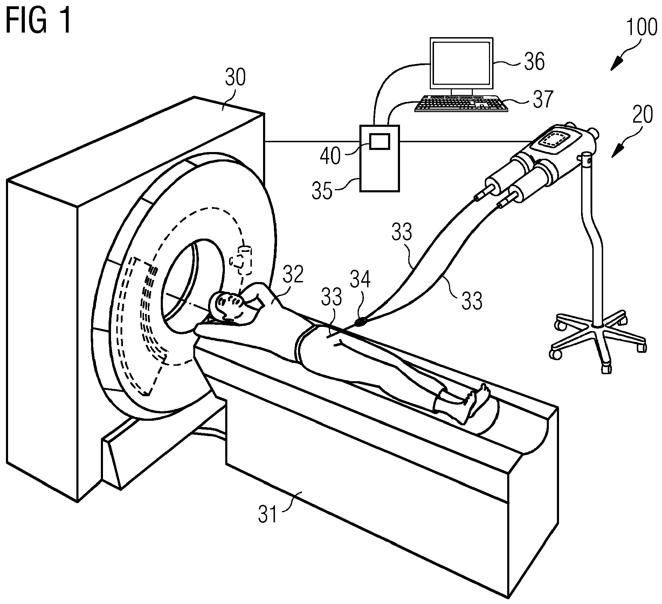

FIG 1 roughly schematically illustrates a computer tomography system (CT system) 100 as an exemplary embodiment of an imaging system according to the invention. The CT system 100 comprises a user terminal 35, 36, 37, a CT device 30, 31 as a medical imaging modality, and a contrast media power injector 20, 40 according to an exemplary embodiment of the invention.

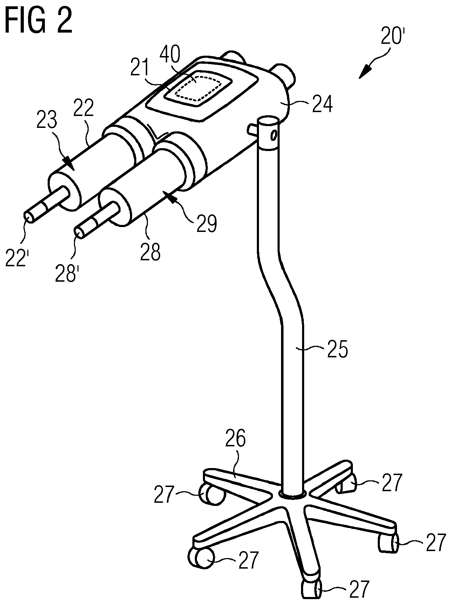

A slightly modified example of a contrast media power injector 20' (in the following abbreviated as power injector) according to an embodiment of the invention is shown in more detail in FIG 2. The power injector 20' comprises a housing 24 for the main components and a stand 25 with a star-shaped five-legged base 26. A wheel 27 is attached to each leg of the base 26. Thus, the power injector 20' can be wheeled and easily transported. As alternatives to the embodiment shown in FIG 2, it is also possible to provide the injector with jointed arms for movability and mount it on a wall, on a ceiling, or on the imaging modality.

A touch display 21 is mounted in the housing 24 and forms part of its surface. The touch display 21 provides a user with information about the injection control settings and is also suitable to enter user input, as described in detail below.

Furthermore, the power injector 20' comprises two syringes 22, 28, which are held by one side of the housing 24 and arranged extending beyond it. Each syringe 22, 28 has a nozzle 22', 28' pointing away from the housing 24. The first syringe 22 forms the first vessel according to the invention and is filled with highly concentrated iodinated contrast medium 23. The second syringe 28 forms the second vessel according to the invention and is filled with a saline solution 29.

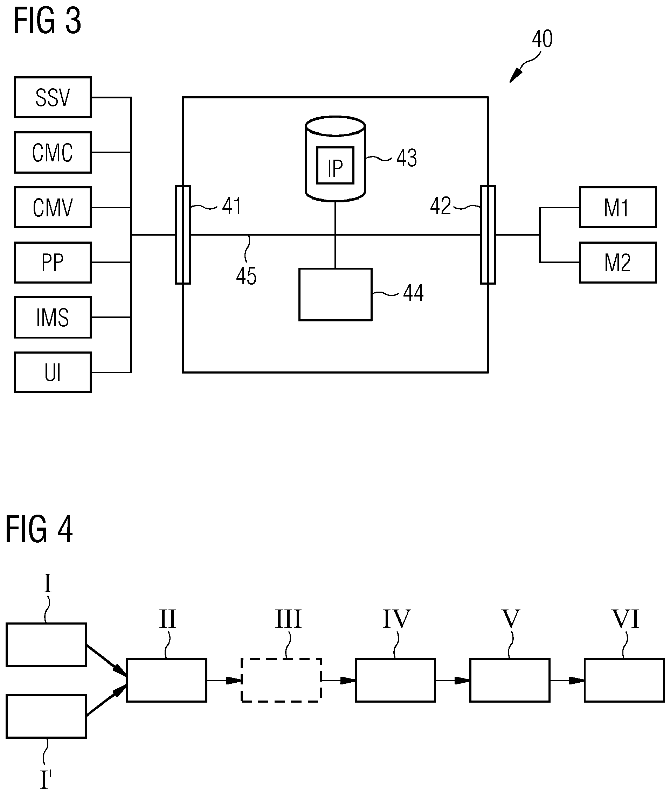

Not shown is a plunger in each of the syringes 22, 28, which is driven by a motor M1, M2 (see FIG 3). As the plunger moves into the volume of the syringe 22, 28 the substance 23, 29 is released from the interior of the syringe 22, 28.

Inside the housing 24 a controller 40 (indicated in dashed lines) according to the invention is arranged. The controller 40 is described in detail below referring to FIG 3. It collects input from the touch-display 21 and controls the motors driving the release of substances 23, 29 from the syringes 22, 28.

The only difference between the power injector 20' of FIG 2 and the power injector 20 of FIG 1 is in the location of the controller 40. The power injector 20' of FIG 2 has the controller 40 integrated inside the housing 24. In contrast, the power injector 20 of FIG 1 utilises an external controller 40 located in a computing unit 35 that is connected to the other parts of the power injector 20. Now back in FIG 1, the syringes 22, 23 of the power injector 20 are interconnected via tubing 33 and a 3-way valve 34. The third passage of the 3-way valve 34 is connected via additional tubing 33 and a suited port to a patient 32 as examination object. This connection to the patient 32 is established by medical personnel and not part of the invention.

The user terminal 35, 36, 37 comprises a computing unit 35, a monitor 36 and a keyboard 37 as input device for user input. In the computing unit 35, which has the necessary storage and processing means as well the interfaces needed for the connections, the controller 40 according to one embodiment of the invention is realised. The connections can be wired as shown or wire-less, for example, via serial port, Ethernet, W-LAN, Bluetooth®, ZigBee or the like.

Apart from the connection to the power injector 20, the computing unit 35 is connected to the CT device 30, 31. The CT device comprises a CT unit 30, the basic structure and function of which are known to the skilled person, and a patient table 31 on which the patient 32 can be positioned in relation to the CT unit 30.

The contrast media power injector 20, 20' is depicted in FIG 1 and FIG 2 with the first vessel 22 and the second vessel 28 realised as syringes 22, 28. It is, however, obvious for a skilled person that the first vessel 22 and the second vessel 28 can also be realised as bottles or other containers, wherein pumps provide for the controlled release of the substances 23, 29. Alternatively, the controller 40 can be integrated directly into the CT unit 30 and no separate computing unit 35 is needed.

FIG 3 shows a block diagram as an example for the realisation of a controller 40 according to the invention. The controller comprises an input interface 41, an output interface 42, storage means 43, processor means 44 and a central bus 45, which connects the other components 41, 42, 43, 44. The storage means 43 may be realised, for example, as a flash drive, EEPROM, HDD SSD or the like. The processor means 44 may be realised, for example, as a common processor, a microprocessor or the like.

Apart from the general commands for controlling the power injector 20, 20' according to the invention the storage means 43 can hold data of various injection protocols IP. The injection protocols IP depend on the patients 32 needs and can be retrieved or modified accordingly.

The input interface receives various inputs SSV, CMC, CMV, PP, IMS, UI and others not shown. The saline solution volume SSV, the contrast medium concentration CMC and the contrast medium volume CMV can, for instance, be entered by scanning barcodes printed on the respective vessels 22, 23. The patient parameters PP can, for example, be imported from an electronic patient record, e.g. from an RIS-System. The controller can receive image modality control signals IMS from a central controller or directly from the imaging modality 30 to coordinate the injection procedure with the imaging procedure. Of course, by means of an additional user input UI, the previous inputs SSV, CMC, CMV, PP, IMS, IP can be changed or further inputs can be made.

On a basis of the suited stored and input data SSV, CMC, CMV, PP, IMS, UI, IP the processor means 44 generates or modifies the predefined injection protocol. For this the required iodine delivery DR rate and injection flow rate FR are determined from the patient parameters PP. During the actual injection procedure the processor means 44 generates control signals. The control signals are send via the output interface 42 in order to control the motors M1, M2 and thus to control the release of substance 23, 29 from the respective syringe 22, 28.

FIG 4 depicts a flow chart of an exemplary method according to the invention. In a preparation step I, the first syringe 22 is provided filled with iodinated contrast medium 23. The contrast medium 23 has a high concentration, preferably at least 350 mgI/mL, more preferably at least 370 mgI/mL, even more preferably at least 390 mgI/mL, most preferably at least 400 mgI/mL. The first syringe 22 is attached to the housing 24 of the power injector 20, 20'.

In preparation step I', the second syringe 28 is provided filled with saline solution 29. The second syringe 23 is also attached to the housing 24 of the power injector 20, 20'.

In steps I and I', the saline solution volume SSV and the saline solution concentration, the contrast medium concentration CMC and the contrast medium volume CMV are entered by scanning barcodes or by a manual user input. Depending on the design of the injector, these parameters can additionally be sent to the imaging modality for coordinating the injection and the imaging procedure.

In a preparation step II, the predefined injection protocol IP is generated or modified by the processor means according to the stored and input data SSV, CMC, CMV, PP, IMS, UI, IP.

For example, for a contrast medium concentration CMC of 370 mg iodine per ml, a desired iodine delivery rate DR of 1.2 g/s and an injection flow rate FR of 4 mL/s. The physical relationship between the parameters is as follows:

$$CMR = DR / CMC / FR$$

In order to obtain the iodine delivery rate DR the contrast medium to saline solution ratio CMR would have to be set to a factor of 0.81. For a fixed total flow rate FR of 4 mL/s, this directly results in the volume flow rates for the contrast medium (FR * CMC = 3.24 ml/s) and the saline solution (FR * (1-CMC) = 0.76 ml/s).

The tubing 33 is connected to the syringes 22, 28. If applicable, pre-run of saline solution 29 from the second syringe 28 through the tubing 33 is triggered.

In a preparation step III, which is not encompassed by the invention as indicated by dashed lines, the tubing 33 is connected to the patient 32 via a suited access or port.

In step IV, the actual injection procedure begins. Therefore, the following steps are timed with the imaging procedure. Step IV is the first injection step. The controller 40 controls the motors M1, M2 of the syringes 22, 28 in such a way that the contrast medium 23 and saline solution 29 are released with the respective flow rates given above. At the 3-way valve they mix to a combined solution which is required for the corresponding imaging procedure. For example, the above mentioned flow rates are applied for 25 s in order to inject a total volume of the combined solution of 100 ml. The period shortly after this first injection step is the early phase, i.e. arterial phase. The contrast is enhanced predominantly in the blood vessels. Therefore the imaging procedure focuses on these regions.

Step V is a second injection step during which the power injector 20, 20' is controlled in such a way that a combined solution with a lower second effective concentration of contrast medium 23 is injected to the patient 32. The decrease of iodine concentration for the second injection phase (if required), in general, depends on the diagnostic protocol. For example, if a reduction of first effective concentration to the second effective concentration by another 50% (resulting in an IR of 0.6 g/s) is desired, this can be achieved by applying a CMR of 0.405 while holding the total FR constant.

In step VI, only saline solution 29 is injected as a saline chaser with a flow rate of 4 ml/s for a duration of 10 s in order to release a total volume of 40 ml.

Although the present invention has been disclosed in the form of preferred embodiments and variations thereon, it will be understood that numerous additional modifications and variations could be made thereto without departing from the scope of the invention. For instance, although only two vessels are described to be part of the contrast media power injector in the above, contrast media power injector can also comprise more vessels for substances to be injected. For the sake of clarity, it is to be understood that the use of "a" or "an" throughout this application does not exclude a plurality, and "comprising" does not exclude other steps or elements. The mention of a "device" or a "system" does not preclude the use of more than one device or system.

**Claims**

1. A controller (40) for a contrast media power injector (20, 20') comprising a first vessel (22) filled with contrast medium (23) at a concentration that is higher than the required concentration for a first injection step (IV); and a second vessel (28) filled with saline solution (29); wherein the controller (40) is realised to release controlled amounts of contrast medium (23) and saline solution (29) to obtain a combined solution with the concentration required for the first injection step (IV).

2. A controller according to claim 1, which is designed to control the release of contrast medium (23) and saline solution (29) to a solution with a concentration to be injected in a second injection step (V).

3. A controller according to claim 1 or 2, which is designed to control the release of saline solution (29) after the injection of the contrast medium (23).

4. A controller according to one of the preceding claims, which controls the release of contrast medium (23) and saline solution (29) according to a predefined injection protocol (IP).

5. A controller according to claim 4, wherein the injection protocol is predefined according to patient parameters (PP).

6.  A controller according to one of the preceding claims, wherein the contrast medium (23) is an iodinated contrast medium (23).

7.  A controller according to claim 6, wherein the concentration of iodine in the contrast medium (23) is at least 350 mgI/mL, preferably at least 370 mgI/mL, more preferably at least 390 mgI/mL, most preferably at least 400 mgI/mL.

8.  A contrast media power injector (20, 20') comprising

    - a first vessel (22) filled with contrast medium (23) at a higher concentration than a concentration to be injected in a first injection step (IV);
    - a second vessel (28) filled with saline solution (29) and
    - a controller (40) according to one of the claims 1 to 7.

9.  A contrast media power injector (20, 20') according to claim 8, wherein the first vessel (22) and/or the second vessel (28) are syringes.

10. A contrast media power injector according to claim 8, wherein the first vessel (22) and/or the second vessel (28) are bottles.

11. An imaging system (100) comprising an imaging modality (30) and a contrast media power injector (20) according to one of the claims 8 to 10.

12. A method for controlling a contrast media power injector (20, 20') comprising a first vessel (22) filled with contrast medium (23) at a concentration that is higher than the required concentration for a first injection step (IV); and a second vessel (28) filled with saline solution (29), which method comprises the step of

    - releasing controlled amounts of contrast medium (23) and saline solution (29) to obtain a combined solution with the concentration required for the first injection step (IV) .

13. A computer program product comprising a computer program that is directly loadable into a controller (40) of a contrast media power injector (20, 20'), which comprises program elements for performing steps of the method according to claim 12 when the computer program is executed by the controller (40).

14. A computer-readable medium on which is stored program elements that can be read and executed by a computer unit in order to perform steps of the method according to claim 12 when the program elements are executed by the computer unit.

FIG 1

FIG 2

FIG 3

SSV

CMC

CMV

PP

IMS

UI

41 ~

45

42 ~

IP ~ 43

~ 44

40

M1

M2

FIG 4

I

I'

II

III

IV

V

VI

Europäisches
Patentamt

European
Patent Office

Office européen
des brevets

# EUROPEAN SEARCH REPORT

**Application Number**

EP 19 21 2856

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/046259 A1 (BAYER HEALTHCARE LLC [US]) 7 March 2019 (2019-03-07) * paragraphs [0074], [0099]; figures 3,7B * | 1-14 | INV. A61M5/00 A61M5/145 A61M5/168 |
| X | WO 2018/190367 A1 (NEMOTO KYORINDO CO LTD [JP]) 18 October 2018 (2018-10-18) * paragraphs [0071], [0074], [0106] - [0108]; figures 2-4 * | 1-14 | ADD. A61B6/00 |
| X | WO 2014/168210 A1 (NEMOTO KYORINDO CO LTD [JP]) 16 October 2014 (2014-10-16) * paragraph [0181]; figures 4D, 4F * | 1-14 | |
| X | CN 109 602 970 A (NANJING GANKONGTONG CHEMICAL PRODUCT OPERATION PORTION) 12 April 2019 (2019-04-12) * paragraphs [0013] - [0016]; figures 1-5 * | 1-14 | |
| X | US 2004/010229 A1 (HOUDE ERIC [US] ET AL) 15 January 2004 (2004-01-15) * paragraphs [0019], [0020], [0026]; figure 1 * | 1-14 | TECHNICAL FIELDS SEARCHED (IPC) A61M A61B |

The present search report has been drawn up for all claims

| Place of search | Date of completion of the search | Examiner |
|---|---|---|
| Munich | 5 June 2020 | Krassow, Heiko |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 19 21 2856

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2020

| Patent document cited in search report | | | Publication date | Patent family member(s) | | | Publication date |
|---|---|---|---|---|---|---|---|
| WO 2019046259 | A1 | | 07-03-2019 | AU | 2018326378 | A1 | 02-01-2020 |
| | | | | CA | 3067624 | A1 | 07-03-2019 |
| | | | | CN | 110809483 | A | 18-02-2020 |
| | | | | WO | 2019046259 | A1 | 07-03-2019 |
| WO 2018190367 | A1 | | 18-10-2018 | CN | 110753562 | A | 04-02-2020 |
| | | | | JP | WO2018190367 | A1 | 27-02-2020 |
| | | | | US | 2020030524 | A1 | 30-01-2020 |
| | | | | WO | 2018190367 | A1 | 18-10-2018 |
| WO 2014168210 | A1 | | 16-10-2014 | JP | 6338190 | B2 | 06-06-2018 |
| | | | | JP | 2018114351 | A | 26-07-2018 |
| | | | | JP | WO2014168210 | A1 | 16-02-2017 |
| | | | | WO | 2014168210 | A1 | 16-10-2014 |
| CN 109602970 | A | | 12-04-2019 | NONE | | | |
| US 2004010229 | A1 | | 15-01-2004 | AT | 458513 | T | 15-03-2010 |
| | | | | AU | 2003261125 | A1 | 02-02-2004 |
| | | | | CA | 2487496 | A1 | 22-01-2004 |
| | | | | EP | 1521606 | A1 | 13-04-2005 |
| | | | | JP | 4663320 | B2 | 06-04-2011 |
| | | | | JP | 2005532140 | A | 27-10-2005 |
| | | | | US | 2004010229 | A1 | 15-01-2004 |
| | | | | WO | 2004006994 | A1 | 22-01-2004 |

EPO FORM P0459

For more details about this annex : see Official Journal of the European Patent Office, No. 12/82